(19) Europäisches Patentamt
European Patent Office
Office européen des brevets

(11) Numéro de publication: **0 224 531**
**B1**

(12) **FASCICULE DE BREVET EUROPEEN**

(45) Date de publication du fascicule du brevet: **28.12.88**

(21) Numéro de dépôt: **86903409.0**

(22) Date de dépôt: **27.05.86**

(86) Numéro de dépôt international: **PCT/FR 86/00178**

(87) Numéro de publication internationale: **WO 86/07045 (04.12.86 Gazette 86/26)**

(51) Int. Cl.⁴: **C 01 B 35/10**, B 01 J 29/04, C 07 C 1/20, C 07 C 5/373, C 07 C 2/08, C 10 G 11/05

(54) **ZEOLITES DU TYPE BOROSILICATE CONTENANT DU FLUOR.**

(30) Priorité: **28.05.85 FR 8507978**

(43) Date de publication de la demande: **10.06.87 Bulletin 87/24**

(45) Mention de la délivrance du brevet: **28.12.88 Bulletin 88/52**

(84) Etats contractants désignés: **AT BE DE GB IT NL SE**

(56) Documents cités:
**FR-A- 2 429 182**
**FR-A- 2 526 414**
**GB-A- 2 062 603**
**US-A- 4 269 813**
**US-A- 4 285 919**

(73) Titulaire: **INSTITUT FRANCAIS DU PETROLE, 4, Avenue de Bois-Préau, F-92502 Rueil-Malmaison (FR)**

(72) Inventeur: **GUTH, Jean-Louis, 59, rue Bellevue Brunstatt, F-68200 Mulhouse (FR)**
Inventeur: **KESSLER, Henri, 17, rue de la Forêt, F-68270 Wittenheim (FR)**
Inventeur: **WEY, Raymond, 3, rue de Alfred Werner, F-68093 MulhouseCedex (FR)**
Inventeur: **FAUST, Anne-Catherine, 23, rue de Bourgogne, F-68100 Mulhouse (FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un nouveau procédé de synthèse de zéolites du type borosilicate, appelés Borozéosilites, les produits obtenus par ce procédé ainsi que leurs applications en adsorptions et en catalyse.

Les zéolites sont des tectosilicates cristallisés. Les structures sont constituées par des assemblages de tétraèdres $TO_4$ formant une charpente tridimensionnelle par la mise en commun des oxygènes. Dans les zéolites du type aluminosilicate qui sont les plus communes, T représente le silicium tétravalent ainsi que l'aluminium trivalent. Les cavités et canaux de dimensions moléculaires de cette charpente accueillent les cations compensant le déficit de charge lié à la présence de l'aluminium trivalent dans les tétraèdres. On connaît aussi quelques rares zéolites où le silicium est remplacé par du germanium tétravalent. De même des éléments trivalents comme le gallium et plus rarement le bore ou le béryllium, peuvent se substituer à l'aluminium.

D'une manière générale, la composition des zéolites peut être représentée par la formule brute $M_{2/n}O$; $Y_2O_3$; $xZO_2$ à l'état déshydraté et calciné. Z et Y représentent respectivement les éléments tétravalent et trivalent des tétraèdres $TO_4$; M représente un élément électropositif de valence n tel qu'un alcalin ou alcalino-terreux et constituant les cations de compensation; x peut varier de 2 à théoriquement l'infini auquel cas la zéolite est une silice.

Chaque type de zéolite possède une structure poreuse distincte. La variation des dimensions et formes des pores d'un type à l'autre entraîne des changements des propriétés adsorbantes. Seules les molécules ayant certaines dimensions et formes sont capables d'entrer dans les pores d'une zéolite particulière. En raison de ces caractéristiques remarquables les zéolites conviennent tout particulièrement pour la purification ou la séparation de mélanges gazeux ou liquides comme par exemple la séparation d'hydrocarbures par adsorption sélective.

La composition chimique avec en particulier la nature des éléments présents dans les tétraèdres $TO_4$ et la nature des cations de compensation échangeables est également un facteur important intervenant dans la sélectivité de l'adsorption et surtout dans les propriétés catalytiques de ces produits. Ils sont utilisés comme catalyseurs ou supports de catalyseurs dans le craquage, le reformage et la modification d'hydrocarbures ainsi que dans l'élaboration de nombreuses molécules.

De nombreuses zéolites existent dans la nature: ce sont des aluminosilicates dont les disponibilités et propriétés ne répondent pas toujours aux exigences des applications industrielles. De ce fait, la recherche de produits ayant des propriétés nouvelles a conduit à la synthèse d'une grande variété de zéolite essentiellement du type aluminosilicate. Parmi les nombreux exemples de ce type on peut signaler: la zéolite A (brevet US n° 2 882 243), la zéolite X (brevet US n° 2 882 244), la zéolite Y (brevet US n° 3 130 007), la zéolite L (brevet français n° 1 224 154), la zéolite T (brevet français n° 1 223 775), la zéolite ZSM5 (brevet US n° 3 702 886), la zéolite ZSM12 (brevet US n° 3 832 449), la zéolite ZSM48 (brevet européen n° 0 015 132).

Par contre, les préparations de zéolites contenant du bore à la place de l'aluminium dans les tétraèdres de la charpente ont été très peu nombreuses (R.M. Barrer «Hydrothermal Chemistry of Zéolites», Academic Press, 1982, p. 283-290; M. Taramasso, G. Perego et B. Notari in «Proceedings of the 5th International Conference on Zeolites» Ed. L.V. C. Rees, p. 40, Heyden, London 1980). Parmi les quelques brevets revendiquant l'incorporation de bore dans la structure de la zéolite, on peut citer les brevets français n° 1 412 923, n° 2 429 182 et n° 2 526 414, le brevet européen n° 0 046 504, le brevet US 4 269 813 et le brevet BE 880 858 (=US-A-4 285 919).

De manière générale, les zéolites sont préparées par cristallisation hydrothermale de mélanges réactionnels contenant des sources d'oxydes ou hydroxydes de métaux alcalins ou alcalino-terreux, de silice, et d'oxydes d'éléments comme l'aluminium pouvant remplacer le silicium dans les tétraèdres. Dans certains cas on ajoute une espèce organique (amine ou ammonium quaternaire). Ces milieux sont caractérisé par un pH basique se situant généralement au-dessus de 10. On admet que cette concentration en ions $OH^-$ facilite la cristallisation de la zéolite en assurant la dissolution des sources de silice, et éventuellement des oxydes amphotères comme l'alumine, ainsi que le transfert des espèces solubles obtenues sur la zéolite en voie de formation. Bien que la présence d'espèces organiques, en particulier des cations dérivés de bases organiques, se traduise généralement par une augmentation de la teneur en silice dans la charpente, on pense actuellement qu'elles interviennent directement dans la formation de la structure de certaines zéolites.

Cette méthode de synthèse des zéolites présente de nombreux inconvénients surtout si on veut remplacer l'aluminium par du bore. En effet, si l'incorporation d'aluminium ou de gallium dans les tétraèdres $TO_4$ de la charpente est facile en milieu basique, il semble que dans ces mêmes conditions l'incorporation du bore soit beaucoup plus difficile. D'autre part, dans un milieu basique la plupart des zéolites synthétisées sont métastables et on risque l'apparition, au cours de la préparation, de phases solides plus stables mais non désirées. Cette difficulté ne fait que s'accroître lorsque les quantités préparées augmentent, c'est-à-dire lorsqu'on passe de l'échelle laboratoire à l'échelle industrielle. De plus, ces zéolites métastables ne sont obtenues que grâce à une forte sursaturation en espèces actives dans le milieu hydrothermal, ce qui produit une nucléation rapide et par conséquent conduit à des cristaux de zéolites de petite taille. Les dimensions moyennes se situent dans la zone du micromètre. L'obtention de cristaux avec des dimensions de quelques dizaines de micromètres est difficile, donc rare. Or, dans certaines applications d'échange d'ions, d'adsorption ou de catalyse, il serait intéressant de pouvoir travailler avec des cristaux de grandes tailles ce qui, par exemple, permettrait d'éviter le conditionnement des zéolites par agglomération avec tout les inconvénients.

De nombreuses applications, en particulier dans la catalyse acide nécessitent des zéolites sous une forme protonée et complètement débarrassée de leurs cations de compensation alcalins ou alcalino-terreux introduits lors de la synthèse. On peut y accéder par des procédés d'échange d'ions répétés et longs avec des cations $NH_4^+$ suivis de calcination pour les décomposer en cations $H^+$. Cette étape d'échange d'ions pourrait être éliminée si on pouvait entièrement remplacer les cations alcalins ou alcalino-terreux par des cations $NH_4^+$ lors de la synthèse. Or, ceci n'est pas possible lorsque le pH dépasse sensiblement 10, $NH_4^+$ étant dans ces conditions transformé en $NH_3$. D'autre part, les synthèses effectuées à des pH où le cation $NH_4$ est stable sont difficiles et longues à cause de la faible solubilité des sources de silice à ces bas pH.

L'invention a donc pour objet un nouveau procédé de préparation de zéolites du type borosilicate appelées Borozéosilites où les inconvénients cités précédemment n'existent plus. Le nouveau procédé s'applique à la préparation de nouvelles zéolites de la famille des pentasils et s'apparentant aux zéolites du type ZSM5 tout en se distinguant de ces dernières par des particularités des diagrammes de diffraction des rayons X et des compositions chimiques. Les nouvelles zéolites ont également des propriétés particulières en adsorption et en catalyse.

La demanderesse a en effet trouvé que les différents inconvénients liés aux méthodes de préparation de zéolites du type borosilicate en milieu basique disparaissent quand on effectue les synthèses dans des milieux aqueux avec un pH inférieur à 10,5 et contenant des anions fluorure. Ainsi les cations dérivés des métaux alcalins ou alcalino-terreux peuvent être remplacés par des cations $NH_4^+$ avec tous les avantages découlant de l'utilisation de ces derniers cations. La solubilisation des sources de silice et de bore est assurée par les ions fluorure qui constituent l'agent mobilisateur et qui remplacent ainsi les ions hydroxyle des milieux basiques. Dans ces conditions, on peut obtenir des cristaux de zéolites du type borosilicate ayant des rapports molaires $SiO_2/B_2O_3$ compris entre 20 et 1000 dont les dimensions peuvent être fixées en fonction des différents paramètres de synthèse (concentration des réactifs, température, durée) entre quelques fractions et quelques centaines de micromètres.

Sous son aspect général, le procédé selon l'invention pour l'obtention de zéolites du type borosilicate de la famille des pentasils consiste:

a) à former un mélange réactionnel ayant un pH inférieur à 10,5 et comprenant notamment de l'eau, une source de silice, une source d'oxyde de bore, une source d'agent mobilisateur contenant des ions fluorure et une source d'agent structurant pouvant fournir des cations organiques, par exemple les cations tétrapropylammonium ($TPA^+$) et tétrapropylphosphonium ($TPP^+$);

b) à chauffer le milieu réactionnel formé dans l'étape a) à une température inférieure à 230°C jusqu'à l'obtention d'un composé cristallin;

c) à calciner le composé obtenu à une température supérieure à 450°C, par exemple 450-1000°C.

Le pH du milieu réactionnel, inférieur à 10,5, peut être obtenu soit directement à partir de l'un ou plusieurs des réactifs mis en œuvre, soit par l'ajout d'un acide, d'une base, d'un sel acide, d'un sel basique ou d'un mélange tampon complémentaire.

De nombreuses sources de silice peuvent être utilisées. On peut citer les silices sous formes d'hydrogels, d'aérogels, de suspensions colloïdales, les silices résultant de la précipitation à partir de solutions de silicates solubles ou de l'hydrolyse d'esters siliciques comme $Si(OC_2H_5)_4$ ou de complexes comme $Na_2SiF_6$, les silices préparées par des traitements d'extraction et d'activation de composés cristallisés naturels ou synthétiques comme les silicates d'aluminium, les aluminosilicates, les argiles.

Parmi les sources d'oxyde de bore, on choisira de préférence l'acide borique $H_3BO_3$ ou l'anhydride borique $B_2O_3$. Mais des sels comme le borax, le tétraborate d'ammonium ou des molécules hydrolysables comme $BF_3$, $BCl_3$ et les esters de l'acide borique, comme le triéthylborate, conviennent également.

Au lieu de partir de sources séparées d'oxydes de bore et de silice on peut aussi prendre des sources où les deux oxydes sont combinés comme par exemple les verres borosilicates ou des borosilicates cristallisés.

Les sources de silice et d'oxyde de bore peuvent être engagées sous forme soluble ou de solides pulvérulents mais également sous des formes massives comme des verres ou des poudres agglomérées (extrudés ou pastilles). Ces derniers conditionnements permettent d'obtenir selon le procédé de l'invention des zéolites du type Borozéosilites déjà agglomérées.

Les anions fluorure $F^-$ peuvent être introduits sous forme de sels de fluor comme NaF, $NH_4F$, $NH_4HF_2$, TPA-F, TPP-F ou de molécules ($SiF_4$, $BF_3$) ou de complexes ($Na_2SiF_6$, $NH_4BF_4$) hydrolysables. Le fluorure d'ammonium ou le fluorure d'ammonium acide sont des sels préférés car ils permettent l'obtention d'une zéolite apparentée à la ZSM5 facile à transformer dans la forme protonée sans qu'il y ait lieu de procéder à des réactions d'échange d'ions.

Les cations organiques sont de préférence des cations tétrahydrocarbylammonium ou tétrahydrocarbyl phosphonium, hydrocarbyl étant avantageusement alkyl.

Ces cations sont ajoutés de préférence sous forme de leurs sels. Mais ils peuvent également être générée in situ, par exemple à partir de tripropylamine ou de tripropylphosphine et d'un halogénure de propyle dans le cas des cations $TPA^+$ et $TPP^+$.

D'autres agents structurants ou chélatants peuvent être utilisés comme reconnu dans l'art antérieur, notamment des composés ayant des fonctions amine, cétone, alcool, acide, par exemple des aminoalcools, des amino-acides, des polyalcools ou des amines tertiaires.

Les acides ou sels acides, les bases ou sels basiques ajoutés éventuellement en complément pour amener le pH du milieu à la valeur désirée peuvent être choisis parmi les acides courants comme HF, HCl, $HNO_3$, $H_2SO_4$, $CH_3COOH$ ou les sels acides comme $NH_4HF_2$, $KHF_2$, $NaHSO_4$, $KHSO_4$, $NaH_2PO_4$; les bases courantes comme $NH_4OH$, NaOH, KOH,

ou les sels basiques courants comme $NaHCO_3$, $Na_2CO_3$, $CH_3COONa$, $Na_2S$, NaHS ou les mélanges tampons comme ($CH_3COOH$, $CH_3COONa$), ($NH_4OH$, $NH_4Cl$).

La morphologie, la taille et la cinétique de formation des cristaux de zéolites obtenus selon le procédé de l'invention peuvent être modifiées par l'introduction dans le milieu réactionnel de sels complémentaires comme NaCl, KCl, $NH_4Cl$, $Na_2SO_4$ et (ou) de cristaux (broyés ou non) apparentés aux zéolites faisant l'objet de cette demande.

Le premier stade de la préparation des nouveaux borosilicates apparentés aux zéolites du type ZMS5 consiste à chauffer à une température comprise entre 60 et 230°C, et de préférence entre 80 et 210°C, un mélange réactionnel dont la composition est avantageusement définie par les valeurs de pH et de rapports figurant dans le tableau I ci-après:

TABLEAU I

| | | Valeurs larges | Valeurs préférées |
|---|---|---|---|
| 1 | pH | 1,5-10,5 | 3-9,5 |
| 2 | rapport molaire $SiO_2/B_2O_3$ | 0,5-800 | 1,5-400 |
| 3 | rapport molaire $F^-/SiO_2$ | 0,04-4 | 0,06-1,5 |
| 4 | rapport molaire $TPA^+$ ou $TPP^+/SiO_2$ | 0,02-1 | 0,06-0,75 |
| 5 | rapport molaire $H_2O/SiO_2$ | 3-200 | 4-80 |
| 6 | rapport molaire sel complémentaire/$SiO_2$ | 0-4 | 0-0,5 |
| 7 | rapport pondéral cristaux zéolite/$SiO_2$ | 0,01-0,10 | 0,02-0,04 |

Les contraintes de composition du mélange réactionnel correspondant aux valeurs définies par les paramètres de synthèse des lignes numérotées de 1 à 5 du tableau I sont particulièrement avantageuses pour l'obtention des nouvelles zéolites faisant l'objet de la présente demande.

Les contraintes de compositions correspondant aux valeurs définies pour les paramètres de synthèse des lignes numérotées 6 et 7 du tableaux I sont facultatives et ne sont utilisées que si on veut agir sur la morphologie et la taille des cristaux ou sur le cinétique de la réaction de cristallisation.

Il est bien entendu que n'importe quelle combinaison des différentes valeurs des paramètres de synthèse telles qu'elles figurent dans le tableau I ne donnera pas nécessairement un résultat satisfaisant. Comme le montreront les exemples qui suivront, certaines combinaisons sont préférables.

Le chauffage du mélange réactionnel se fait de préférence dans un autoclave revêtu de polytétrafluoroéthylène. Suivant la composition et la température de chauffage, la durée se situe généralement entre 8 heures et 400 heures. Lorsque la cristallisation est achévée le solide obtenu est filtré et lavé à l'eau désionisée.

Le deuxième stade de la préparation consiste à chauffer les cristaux obtenus dans le stade précédent à une température supérieure à 450°C de façon à détruire et à chasser les espèces organiques liées à l'agent structurant, présentes dans les pores de la zéolite.

Les Borozéosilites, apparentées aux zéolites du type ZSM5 selon l'invention, sont caractérisées après calcination, par les formules exprimées en oxydes

$$(0\text{-}1)\ M_{2/n}O;\ B_2O_3;\ xSiO_2$$

où x peut varier de 20 à 1000 et où M représente les cations de compensation de valence n. M représente le proton $H^+$ résultant de la décomposition des cations azotés, par exemple $NH_4^+$, tetraalkylammonium$^+$ ou tétraalkylphosphonium$^+$, et (ou) les cations non-décomposables issus du milieu réactionnel, par exemple des cations alcalins ou alcalinoterreux ou d'autres métaux précisés ci-après. On peut introduire dans les zéolites par des méthodes d'échange d'ions bien connues les élémentes du tableau périodique dont les cations peuvent être préparés en milieu aqueux. On citera à titre d'exemple les cations alcalins, alcalino-terreux, les cations de terres rares, $Fe^{II}$, $Fe^{III}$, $Co^{II}$, $Co^{III}$, $Ni^{II}$, $Cu^{II}$, $Zn^{II}$, etc.

L'analyse chimique montre qu'il y a en outre entre 0,04 et 1,6% pondéral d'élément fluor dans les zéolites après calcination.

L'identification des Borozéosilites obtenues selon l'invention se fait de manière commode à partir de leur diagramme de diffraction des rayons X. Ce diagramme de diffraction peut être obtenu à l'aide d'un diffractomètre en utilisant la méthode classique des poudres avec le rayonnement $K\alpha$ du cuivre. A partire de la position des pics de diffraction représentée par l'angle $2\theta$ on calcule, par la relation de Bragg, les équidistances réticulaires $d_{hkl}$ caractéristiques de l'échantillon. L'estimation de l'erreur de mesure $\Delta$ ($d_{hkl}$) sur $d_{hkl}$ se calcule, en fonction de l'erreur absolue $\Delta$ ($2\theta$) affectée à la mesure de $2\theta$, par la relation de Bragg. Une erreur absolue $\Delta$ ($2\theta$) égale à $\pm$ 0,2° est couramment admise. L'intensité relative $I/I_o$ affectée à chaque valeur de $d_{hkl}$ est estimée à partir de la hauteur du pic de diffraction correspondant. On utilise souvent une échelle de symboles pour caractériser cette intensité: FF = très fort, F = fort, mF = moyen à fort, m = moyen, mf = moyen à faible, f = faible, ff = très faible.

Le tableau II contient le diagramme de diffraction des rayons X caractéristiques des zéolites du type Borozéosilites obtenues selon l'invention et calcinées à 550°C. Dans la colonne des $d_{hkl}$ on a représenté les valeurs extrêmes que peuvent prendre les différentes équidistances réticulaires $d_{hkl}$: les variations observées sont essentiellement liées à la nature des cations de composition et au rapport Si/B de la zéolite. Chacune de ces valeurs doit encore être affectée de l'erreur de la mesure $\Delta$ ($d_{hkl}$).

Les Borozéosilites préparées selon l'invention sont

des adsorbants sélectifs. Ainsi ils permettent la séparation des xylènes par adsorption sélective du paraxylène. Mais leur principal intérêt réside dans leur utilisation comme catalyseurs ou support de catalyseurs, purs ou en mélange, pour des transformations très spécifiques de composés organiques variès allant des hydrocarbures à des composés contenant, outre du carbone et de l'hydrogène, de l'oxygène, de l'azote, du soufre, etc.

A titre d'exemple on peut citer pour les hydrocarbures

— l'isomérisation des oléfines, en particulier l'isomérisation des butènes en isobutylène
— la déshydroisomérisation des alcanes, en particulier la déshydroisomérisation du butane en isobutylène
— l'oligomérisation des oléfines
— l'isomérisation des aromatiques comme les xylènes
— l'alkylation, la dismutation et la transalkylation des aromatiques
— la conversion du méthanol en oléfines.

Egalement, à titre d'exemple, on peut citer pour les composés oxygénés la formation d'oléfines légères comme l'éthène ou le propène à partir du méthanol.

Une autre application importante est le craquage des hydrocarbures. Pour cette application on préfère utiliser les borozéosilites en mélange avec un catalyseur zéolithique de craquage, à raison de 0,2 à 10% en poids, de préférence 0,5 à 5% en poids, de borozéosilite, le complément étant le catalyseur zéolithique et une matrice amorphe.

De préférence le catalyseur de craquage est une zéolithe Y, par exemple:
la forme $NH_4$
la forme stabilisée dite ultrastable (USY)
la forme stabilisée et désaluminée (Deal. USY)
et la forme REY (échangée aux terres rares) avec 0,05-18% en poids de terre rare sous forme d'oxyde ($RE_2O_3$).

TABLEAU II

| $d_{hkl}$ (Å) | $I/I_o$ | $d_{hkl}$ (Å) | $I/I_o$ |
|---|---|---|---|
| 11,25-10,90 | FF | 3,31 -3,28 | ff |
| 10,00- 9,80 | m | 3,26 -3,22 | ff |
| 9,85- 9,61 | f | 3,16 -3,13 | ff |
| 9,10- 8,92 | ff | 3,12 -3,09 | ff |
| 7,50- 7,35 | ff | 3,05 -3,01 | ff |
| 7,13- 6,98 | ff | 2,984-2,953 | ff |
| 6,73- 6,59 | ff | 2,952-2,916 | ff |
| 6,34- 6,24 | f | 2,859-2,841 | ff |
| 6,00- 5,89 | f | 2,780-2,765 | ff |
| 5,90- 5,81 | ff | 2,728-2,708 | ff |
| 5,54- 5,44 | ff | 2,681-2,662 | ff |
| 5,37- 5,24 | ff | 2,654-2,632 | ff |
| 5,17- 5,05 | ff | 2,596-2,578 | ff |
| 5,04- 4,98 | ff | 2,562-2,541 | ff |
| 4,97- 4,91 | ff | 2,513-2,491 | ff |
| 4,89- 4,80 | ff | 2,483-2,464 | ff |
| 4,62- 4,54 | ff | 2,404-2,388 | ff |
| 4,47- 4,41 | ff | 2,392-2,372 | ff |

TABLEAU II (suite)

| $d_{hkl}$ (Å) | $I/I_o$ | $d_{hkl}$ (Å) | $I/I_o$ |
|---|---|---|---|
| 4,36- 4,30 | ff | 2,321-2,300 | ff |
| 4,27- 4,20 | f | 2,290-2,271 | ff |
| 4,09- 4,02 | ff | 2,192-2,174 | ff |
| 4,01- 3,95 | ff | 2,166-2,149 | ff |
| 3,87- 3,82 | m | 2,104-2,088 | ff |
| 3,81- 3,77 | m | 2,074-2,058 | ff |
| 3,75- 3,72 | mf | 2,061-2,048 | ff |
| 3,72- 3,67 | mf | 2,046-2,031 | ff |
| 3,65- 3,60 | f à mf | 2,011-1,990 | f |
| 3,60- 3,56 | f à ff | 1,985-1,966 | f |
| 3,48- 3,44 | ff | 1,946-1,928 | ff |
| 3,44- 3,40 | ff | 1,908-1,891 | ff |
| 3,39- 3,36 | ff | 1,869-1,848 | ff |
| 3,35- 3,31 | ff | | |

Si la plupart de ces réactions peuvent également être catalysées à l'aide des catalyseurs basés sur des zéolites du type ZSM5 contenant de l'aluminium dans les tétraèdres, l'utilisation des zéolites du type Borozéosilite de la présente invention constitue un progrès très important. En effet, l'acidité plus modérée des catalyseurs à base de borosilicate ne conduit pas à des réactions de craquage et de formation de coke dans des proportions aussi importantes que dans les cas des aluminosilicates correspondants. Ceci permet d'assurer une meilleure sélectivité à la réaction choisie et prolonge la durée d'utilisation du catalyseur avant la régénération.

De plus, on notera que les zéolites selon l'invention peuvent être complètement défluorées par traitement hydrothermal de celle-ci à 170°C avec une solution de $NH_4Oh$ et un rapport liquide/solide de 10.

Les exemples qui suivent sont destinés à illustrer l'invention de façon non limitative.

*Exemple 1*

On prépare une solution contenant 1,8 g d'acide borique, 39,9 g de bromure de tétrapropylammonium (TPA Br) et 11,1 g de fluorure d'ammonium dans 270 g d'eau. On mélange cette solution à 21,6 g de silice précipitée en poudre contenant 16% en poids d'eau, commercialisée par la société Merck. La composition molaire du mélange ramenée à 1 mole de silice est la suivante:

$$1\ SiO_2;\ 0,1\ H_3BO_3;\ 0,5\ TPA\ Br;\ 1\ NH_4F;\ 50\ H_2O$$

Le mélange (pH = 7,5) est chauffé pendant 6 jours à 173°C dans un autoclave renfermant un flacon de 1 l en polytétrafluoroéthylène; le pH final est de 7. Le solide obtenu après filtration et lavage à l'eau désionisée pèse 19,8 g. La taille des cristaux de forme prismatique est voisine de 90 × 20 micromètres. La perte de masse par calcination à 550°C à l'air pendant 2 h est de 11,7%.

L'analyse par diffraction des rayons X du produit calciné montre qu'il s'agit d'une Borozéosilite caractérisée par le diagramme de diffraction du tableau II.

L'analyse chimique de la zéolite calcinée donne une teneur en bore de 0,20% et en élément fluor de 0,22% en poids.

*Exemple 1 bis* (comparatif)

De manière à déterminer si la présence d'ion fluorure est nécessaire au processus de cristallisation, on a préparé un mélange réactionnel dans les mêmes conditions que celles de l'exemple 1 sans ajouter, toutefois, $NH_4F$. La composition molaire du mélange ramenée à 1 mole de silice étant la suivante:

1 $SiO_2$; 0,1 $H_3BO_3$; 0,5 TPA Br; 50 $H_2O$

Le mélange a été chauffé pendant 6 jours à 173°C dans un autoclave. Le solide obtenu après filtration et lavage pesait 13 g. L'analyse aux rayons X a montré que ce solide était totalement amorphe.

*Exemple 2*

On prépare un mélange ayant la composition molaire suivante:

1 $SiO_2$; 1 $H_3BO_3$; 0,5 TPA Br; $NH_4F$; 50 $H_2O$

en engageant 3/10 des quantités molaires indiquées.

La même source de silice que dans l'exemple 1 est utilisée.

Le mélange (pH = 7) est chauffé pendant 6 jours à 170°C dans la même type d'autoclave que celui utilisé dans l'exemple 1; le pH final est de 7. Après filtration et lavage on obtient 20 g de solide, les cristaux prismatiques ont une taille de 135 × 10 micromètres.

Après calcination à 550°C pendant 2 h à l'air (perte de masse = 11%) la teneur en bore est de 0,5% et celle en fluor de 0,27% en poids. Le diagramme de diffraction des rayons X du produit calciné correspond à celui du tableau II.

*Exemple 3*

Dans cette préparation on a de plus ajouté 400 mg d'une Borozéosilite broyée résultant d'une autre synthèse et contenant 0,64% de bore en poids. La composition molaire du mélange est:

1 $SiO_2$; 1 $H_3BO_3$; 0,125 TPA Br; 0,125 $NH_4F$; 6 $H_2O$

dont 3/10 des quantités molaires indiquées sont engagées. La source de silice est la même que dans les exemples 1 et 2.

Le chauffage se fait dans un autoclave chemisé de polytétrafluoroéthylène d'une contenance de 250 ml.

Les conditions opératoires sont:

| Durée | Température | pH initial | pH final |
|---|---|---|---|
| 6 jours | 170°C | 7 | 6 |

Une masse de 21 g de cristaux prismatiques (taille: 1 à 2 micromètres) est obtenue après filtration et lavage. Les teneurs pondérales de ce produit en bore et en fluor sont respectivement de 0,85% et de 0,48%. Le produit calciné à 550°C pendant 5 h à l'air présente un diagramme de diffraction des rayons X analogue à celui du tableau II. Son analyse chimique donne un raport molaire Si/B de 18 et une teneur en élément fluor de 0,31% en poids.

*Exemple 4*

Cet exemple de préparation illustre l'utilisation de fluorure de sodium en remplacement du fluorure d'ammonium; par ailleurs on ajoute la moitié de $TPA^+$ sous forme d'une solution aqueuse à 20% de TPAOH.

La composition molaire du mélange employé est de

1 $SiO_2$; 1 $H_3BO_3$; 0,5 TPAOH; 0,5 TPABr;
1 NaF; 44 $H_2O$

On engage 3/100 des proportions molaires; la source de silice est la même que dans les préparations précédentes.

Les conditions opératoires sont les suivantes:

| Durée | Température | pH initial | pH final |
|---|---|---|---|
| 3 jours | 170°C | 10 | 8 |

Un autoclave de 75 ml revêtu de polytétrafluoroéthylène est employé.

Une masse de 2,8 g de zéolite est obtenue après filtration et lavage. Le diagramme de diffraction des rayons X du produit calciné est conforme aux valeurs du tableau II et le rapport molaire Si/B est égal à 10.

*Exemple 5*

Dans cette préparation on utilise une source de silice différente; il s'agit de silice en poudre obtenue par hydrolyse de l'ester $Si(OC_2H_5)_4$.

La composition molaire du mélange réactionnel est la suivante:

1$SiO_2$; 0,1 $H_3BO_3$; 0,5 TPABr; 1 $NH_4F$; 50 $H_2O$

On engage 3/10 de ces quantités molaires. Le chauffage se fait dans le même type d'autoclave que pour les exemples 1 et 2.

Les conditions opératoires sont:

| Durée | Température | pH initial | pH final |
|---|---|---|---|
| 6 jours | 173°C | 6 | 7 |

Après filtration et lavage on obtient 20 g de solide constitué de cristaux prismatiques dont la dimension est de 85 × 15 micromètres environ.

La perte de masse par chauffage à 550°C à l'air pendant 2 h est de 11,2%. Le diagramme de diffraction des rayons X du produit calciné correspond à celui du tableau II. L'analyse chimique de ce produit calciné conduit à un rapport molaire Si/B de 60, la teneur en fluor est de 0,11% en poids.

*Exemple 6*

Dans cette préparation on engage une source de silice pulvérulente obtenue par pyrohydrolyse du tétrachlorure de silicium, commercialisée par la Société Degussa sous le nom «Aerosil». Sa teneur en eau est de 3% en masse environ.

On prépare une solution contenant 37,08 g d'acide borique, 19,97 g de bromure de tétrapropylammonium, 5,55 g de fluorure d'ammonium et 356 g d'eau dans un flacon de polytétrafluoroéthylène de 1 l. La silice (36,12 g) est ajoutée à cette solution sous agitation continuelle.

La composition molaire du mélange ramenée à une mole de silice est:

1 $SiO_2$; 1 $H_3BO_3$; 0,125 TPABr; 0,250 $NH_4F$; 33 $H_2O$

Les conditions de chauffage en autoclave et les valeurs de pH sont:

| Durée | Température | pH initial | pH final |
|---|---|---|---|
| 14 jours | 170°C | 5 | 5 |

Le solide obtenu après filtration et lavage (masse = 38 g) est constitué de cristaux prismatiques de tailles voisines de 80 × 30 micromètres. L'analyse chimique donne des teneurs pondérales en bore et en fluor de 0,50% et 0,18% respectivement. La perte de masse par calcination à 550°C pendant 2 h est de 12,2%. Le diagramme de diffraction de rayons X est conforme au tableau I. Le rapport Si/B trouvé par analyse chimique est de 28, la teneur en fluor est de 0,14% en poids. Par un traitement hydrotermal à 170°C pendant 6 h, avec une solution $NH_4OH$ 0,15 mole/1 et un rapport liquide/solide de 10 on obtient un produit complètement défluoré.

On introduit 0,8% de platine, par échange d'ions avec une solution $Pt(NH_3)_4Cl_2$ suivi d'une réduction par l'hydrogène, dans 1 g de Borozéosilite préalablement calcinée à 550°C. Ce catalyseur bifonctionnel est testé dans un microréacteur porté à 620°C avec un mélange de n-butane et d'hydrogène dans un rapport volumique 1/1 sous une pression de $10^5$ Pa. Avec un $W/F_o$ de 500 $kg.s.mol^{-1}$ (où W est le poids du catalyseur et $F_o$ le débit en mol/s) on obtient 22% d'isobutylène à côté de 28% de n-butane non transformé.

*Exemple 7*

Cet exemple illustre l'utilisation d'un verre borosilicate comme source des éléments silicium et bore. On prépare un verre de composition 0,5 $B_2O_3$; $SiO_2$ par fusion d'un mélange d'anhydride borique et de silice dans un creuset de platine. Le verre est finement broyé (particules inférieures à 40 micromètres). A partir de cette poudre de verre on réalise un mélange dont la composition molaire est la suivante:

1 $SiO_2$; 0,5 $B_2O_3$; 0,125 TPA-Br; 0,25 $NH_4F$; 15 $H_2O$

On engage 1/10 des quantités molaires indiquées dans un autoclave de 75 ml chemisé de polytétrafluoroéthylène et on chauffe le tout à 200°C pendant 8 jours. Après réaction le solide obtenu est filtré et lavé à l'eau désionisée. Après séchage et calcination à 550°C on obtient une Borozéosilite constituée de cristaux dont la taille est égale à 20 × 10 micromètres et dont le rapport Si/B est ègal à 13.

Un test catalytique a été effectué sur 0,5 g de Borozéosilite calcinée disposée dans un microréacteur chauffé à 500°C couplé à un chromatographe en phase gazeuse. De méthanol injecté avec PP'H de 5,5 kg $kg^{-1}$ $H^{-1}$ (P = poids de méthanol, P' = poids de catalyseur, H = heure) est converti à 100% en hydrocarbures contenant 11% d'éthène, 32% de propène et 21% de butène.

*Exemple 8*

On refait la synthèse de l'exemple précédent mais en pastillant préalablement la poudre de verre borosilicate sous forme de pastille de 3 mm de diamètre sans liant. Après réaction à l'autoclave et calcination à 550°C, on obtient des pastilles présentant la valeur de 12 Newton/mm dans un test d'écrasement et formées de cristaux de Borozéosilite ayant un rapport Si/B égal à 11.

*Exemple 9*

Cet exemple illustre la possibilité d'effectuer des synthèses à une température inférieure à 100°C, l'ajout de germes permettant de réduire la durée de cristallisation à quelques jours. On réalise un mélange ayant la composition molaire suivante:

1 $SiO_2$; 0,1 $H_3BO_3$; 0,25 TPABr; 0,8 $NH_4F$; 15 $H_2O$

en utilisant de la silice Aerosil 130. Le mélange réactionnel contient en plus 8% en poids, par rapport au poids de silice engagée, de cristaux d'une Borozéosilite finement broyés. Il est chauffé 16 jours à 90°C dans un flacon en polypropylène. Après filtration et calcination à 550°C on obtient 64 g de cristaux de Borozéosilite ayant un rapport Si/B de 24.

*Exemple 10*

Sur la Borozéosilite de l'exemple 4, on fait passer du propène dans les conditions opératoires suivantes:

— température: 340°C
— pression: 4 MPa
— débit horaire de propène: 1 kg/1 kg de catalyseur.

Dans ces conditions opératoires, le taux de transformation du propène était de 81%.

Le produit liquide soutiré, après séparation du propène non transformé, présentait les caractéristiques suivantes:

| | |
|---|---|
| — densité à 20°C | 0,784 |
| — indice de brome | 76 |
| — distillation ASTM point initial: °C | 78°C |
| 10% volume | 142°C |
| 30% volume | 165°C |
| 50% volume | 206°C |
| 70% volume | 246°C |
| 90% volume | 288°C |
| 95% volume | 300°C |
| Point final: °C | 306°C |

*Exemple 11* Craquage catalytique.

On est parti de 2 catalyseurs conventionnels:

*Catalyseur A*
Zéolithe HY désaluminée dont les caractéristiques physicochimiques sont données ci-dessous.

| % Na (poids) | $SiO_2$ / $Al_2O_3$ (moles) | Diffraction X | | Adsorption % poids |
|---|---|---|---|---|
| | | Cristallinité (%) | Paramètre (nm) | Azote |
| 0,3 | 12 | 90 | 2,445 | 20% |

*Catalyseur B*
obtenu à partir de zéolithe $NH_4Y$ de rapport $SiO_2/Al_2O_3$ = 4,7 échangée avec une solution aqueuse de terres rares renfermant, en poids:

$La_2O_3$ = 57% poids
$CeO_2$ = 15% poids
$Nd_2O_3$ = 21% poids
$Pr_6O_{11}$ = 71% poids

A l'issue de l'échange on obtient une zéolite REY aux terres rares dont la teneur totale en terres rares est de 14,1% poids. Cette zéolithe conserve le rapport $SiO_2/Al_2O_3$ initial de $NH_4Y$, soit 4,7.

*Catalyseur C:* c'est celui obtenu à l'exemple 7.

*Catalyseur D:* c'est celui obtenu à l'exemple 6.

Les catalyseurs A à D ou leurs mélanges entre eux, tels que définis dans le tableau III, sont dispersés à raison de 20% en poids dans une silice amorphe, la granulométrie du mélange résultant se situant entre 40 et 200 microns.

TABLEAU III (% en poids)

| | |
|---|---|
| Catalyseur 1 | 100% catalyseur A |
| Catalyseur 2 | 100% catalyseur B |
| Catalyseur 3 | 90% catalyseur A + 10% catalyseur C |
| Catalyseur 4 | 80% catalyseur A + 20% catalyseur D |
| Catalyseur 5 | 90% catalyseur B + 10% catalyseur C |
| Catalyseur 6 | 100% catalyseur $NH_4(SiO_2/Al_2O_3$ = 4,7, % Na < 1% poids) |

Chacune des poudres ainsi obtenues est soumise au traitement hydrotermique suivant: 8 heures à 770°C sous 1 bar de pression partielle de vapeur d'eau, pour simuler la désactivation que subissent les catalyseurs dans les unités industrielles.

L'aptitude de chaque catalyseur à convertir un distillat sous vide (DSV) est ensuite déterminée dans les conditions suivantes:

— vitesse spatiale massique = 15 $h^{-1}$

— température T = 480°C.

La charge utilisée (DVS) a les caractéristiques suivantes:

— densité 15°C = 0,904
— %poids S = 1,3
— % poids N < 0,1
— Carbone Conradson % = 0,32
— Ni + V (ppm) < 1

ASTM D 1160
PI = 202°C
10% = 307°C
50% = 402°C
90% = 510°C
PF = 585°C

*Comparaison des performances catalytiques.*
Les résultats sont exprimés de la manière suivante:

— conversion de la charge en % poids
— rendement en gaz ($H_2$ + hydrocarbures de $C_1$ à $C_4$)
— rendement en essence $C_5$-220°C
— indice d'octane (Recherche) (NOR) de l'essence
— coke

Le tableau IV présente des résultats.

| Performances Catalytiques % poids | CATALYSEURS 1 | 2 | 3 | 4 | 5 | 6 |
|---|---|---|---|---|---|---|
| Conversion | 71,9 | 73,8 | 72,0 | 72,0 | 73,6 | 70,3 |
| Gaz ($H_2$+$C_1$ à $C_4$) | 18,6 | 18,1 | 19,4 | 20,3 | 18,8 | 17,9 |
| Essence $C_5$-220°C | 49,2 | 50,6 | 48,4 | 47,5 | 49,8 | 48,3 |
| N.O.R. | 91,1 | 89,6 | 91,5 | 92,3 | 90,1 | 90,8 |
| Coke | 4,1 | 5,1 | 4,2 | 4,2 | 5,0 | 4,1 |

Ces résultats montrent qu'une petite quantité de borozéosilite associée à une zéolithe Y classique de craquage catalytique permet d'améliorer substantiellement l'indice d'octane de l'essence. La diminution de rendement en essence qui accompagne l'augmentation d'indice d'octane peut être compensée par l'utilisation des oléfines en $C_4$ (dont le rendement augmente parallèlement à l'indice d'octane) dans une unité d'alkylation, pour former avec l'isobutane un alkylat d'indice d'octane élevé, ou dans une unité d'oligomérisation.

**Revendications**

1. Procédé de synthèse de zéolithes du type borosilicate renfermant du fluor, caractérisé en ce qu'il consiste:

a) à former un milieu réactionnel ayant un pH inférieur à 10,5 et comprenant notamment de l'eau, une source de silice, une source d'oxyde de bore, une source d'ions fluorure et une source d'agent structurant fournissant des cations organiques,

b) à chauffer le milieu réactionnel formé dans l'étape a) à une température inférieure à 230°C jusqu'à l'obtention d'un composé cristallin,

c) à calciner le composé obtenu à une température supérieure à 450°C.

2. Procédé selon la revendication 1, caractérisé en ce que le pH du milieu réactionnel est compris entre 1,5 et 10,5, le rapport molaire fluorure/silice dans ledit mélange réactionnel est compris entre 0,04 et 4, le rapport molaire cation organique/silice est compris entre 0,02 et 1, le rapport molaire eau/silice est compris entre 3 et 200 et le rapport molaire $SiO_2/B_2O_3$ est compris entre 0,5 et 800.

3. Procédé selon la revendication 2, dans lequel le pH est de 3 à 9,5, le rapport molaire fluorure/silice de 0,06 à 1,5, le rapport molaire cation organique/silice de 0,06 à 0,75, le rapport molaire eau/silice de 4 à 80 et le rapport molaire $SiO_2/B_2O_3$ de 1,5 à 400.

4. Procédé selon l'une des revendications 1 à 3, dans lequel la source d'agent structurant est une source pouvant fournir des cations tétraalkylammonium ou tétraalkylphosphonium.

5. Procédé selon l'une des revendications 1 à 3, dans lequel la source d'agent structurant est une source pouvant fournir des cations choisis parmi les cations tétrapropylammonium ($TPA^+$) et tétrapropylphosphonium ($TPP^+$).

6. Procédé selon l'une des revendications 1 à 5, dans lequel la source d'ions fluorure est le fluorure d'ammonium ou le fluorure acide d'ammonium.

7. Procédé selon l'une quelconque des revendications 1 à 6, caractérisé en ce que ledit mélange réactionnel comprend en outre un sel complémentaire choisi dans le groupe formé par NaCl, KCl, $NH_4Cl$ et $Na_2$ $SO_4$, le rapport molaire sel complémentaire/silice étant compris entre 0 et 4.

8. Procédé selon l'une quelconque des revendications 1 à 7, caractérisé en ce que ledit mélange réactionnel comprend des cristaux d'une zéolite du type à obtenir, le rapport pondéral cristaux de zéolite/silice étant compris entre 0,01 et 0,10.

9. Procédé selon l'une quelconque des revendications 1 à 8, caractérisé en ce que le mélange réactionnel est chauffé à une température comprise entre 60 et 230°C pendant une durée qui se situe généralement entre 8 et 400 heures jusqu'à obtention d'un solide cristallin qui est séparé puis calciné à une température supérieure à 450°C, de façon à détruire et à chasser les espèces organiques liées à l'agent structurant, présentes dans les pores de la zéolite.

10. Zéolite cristallisées du type borosilicate, apparentées aux pentasils répondant à la formule:

$$(0\text{-}1)\ M_{2/n}O : B_2O_3 : x\ SiO_2,$$

dans laquelle x peut varier de 20 à 1000, M représente des cations de compensation de valence n, lesdites zéolites contenant 0,04 à 1,6% en poids de fluor et étant en outre caractérisées par le diagramme de diffraction des rayons X du tableau suivant:

| $d_{hkl}$ (Å) | $I/I_o$ | $d_{hkl}$ (Å) | $I/I_o$ |
|---|---|---|---|
| 11,25-10,90 | FF | 3,31 -3,28 | ff |
| 10,00- 9,80 | m | 3,26 -3,22 | ff |
| 9,85- 9,61 | f | 3,16 -3,13 | ff |
| 9,10- 8,92 | ff | 3,12 -3,09 | ff |
| 7,50- 7,35 | ff | 3,05 -3,01 | ff |
| 7,13- 6,98 | ff | 2,984-2,953 | ff |
| 6,73- 6,59 | ff | 2,952-2,916 | ff |
| 6,34- 6,24 | f | 2,859-2,841 | ff |
| 6,00- 5,89 | f | 2,780-2,765 | ff |
| 5,90- 5,81 | ff | 2,728-2,708 | ff |
| 5,54- 5,44 | ff | 2,681-2,662 | ff |
| 5,37- 5,24 | ff | 2,654-2,632 | ff |
| 5,17- 5,05 | ff | 2,596-2,578 | ff |
| 5,04- 4,98 | ff | 2,562-2,541 | ff |
| 4,97- 4,91 | ff | 2,513-2,491 | ff |
| 4,89- 4,80 | ff | 2,483-2,464 | ff |
| 4,62- 4,54 | ff | 2,404-2,388 | ff |
| 4,47- 4,41 | ff | 2,392-2,372 | ff |
| 4,36- 4,30 | ff | 2,321-2,300 | ff |
| 4,27- 4,20 | f | 2,290-2,271 | ff |
| 4,09- 4,02 | ff | 2,192-2,174 | ff |
| 4,01- 3,95 | ff | 2,166-2,149 | ff |
| 3,87- 3,82 | m | 2,104-2,088 | ff |
| 3,81- 3,77 | m | 2,074-2,058 | ff |
| 3,75- 3,72 | mf | 2,061-2,048 | ff |
| 3,72- 3,67 | mf | 2,046-2,031 | ff |
| 3,65- 3,60 | f à mf | 2,011-1,990 | f |
| 3,60- 3,56 | f à ff | 1,985-1,966 | f |
| 3,48- 3,44 | ff | 1,946-1,928 | ff |
| 3,44- 3,40 | ff | 1,908-1,891 | ff |
| 3,39- 3,36 | ff | 1,869-1,848 | ff |
| 3,35- 3,31 | ff | | |

dans lequel $d_{hkl}$ est l'équidistance réticulaire exprimée en angströms (1 angström = $10^{-10}$ mètre), I/Io représente l'intensité relative exprimée comme FF = très fort; F = fort; mF = moyen à fort; m = moyen; mf = moyen à faible; f = faible; ff = très faible.

11. Utilisation des zéolites selon la revendication 10 à titre de catalyseurs ou supports de catalyseurs en particulier pour la transformation du méthanol en oléfines légères, la déhydroisomérisation du n-butane en isobutylène, l'oligomérisation des oléfines et le craquage catalytique.

**Patentansprüche**

1. Verfahren zur Synthese von Zeolithen vom Borsilikattyp, die Fluor enthalten, dadurch gekennzeichnet, dass es besteht aus:

a) Bildung eines Reaktionsmilieus mit einem pH-Wert kleiner als 10,5 und insbesondere Wasser, eine Siliciumdioxidquelle, eine Boroxidquelle, eine Quelle von Fluoridionen und eine Quelle von strukturierendem Mittel, das organische Kationen liefert, umfassend;

b) Erhitzen des in Stufe a) gebildeten Reaktionsmilieus auf eine Temperatur kleiner als 230°C bis zum Erhalten einer kristallinen Verbindung,

c) Kalzinierung der erhaltenen Verbindung bei einer Temperatur grösser als 450°C.

2. Verfahren gemäss Anspruch 1, dadurch gekennzeichnet, dass der pH-Wert des Reaktionsmilieus zwischen 1,5 und 10,5 liegt, das molare Verhältnis Fluor/Siliciumdioxid in genannter Reaktionsmischung zwischen 0,04 und 4 liegt, das molare Verhältnis organisches Kation/Siliciumdioxid zwischen 0,02 und 1 liegt, das molare Verhältnis Wasser/Siliciumdioxid zwischen 3 und 200 liegt und das molare Verhältnis $SiO_2/B_2O_3$ zwischen 0,5 und 800 liegt.

3. Verfahren gemäss Anspruch 2, in welchem der pH-Wert von 3 bis 9,5 ist, das molare Verhältnis Fluor/Siliciumdioxid von 0,06 bis 1,5, das molare Verhältnis organisches Kation/Siliciumdioxid von 0,06 bis 0,75, das molare Verhältnis Wasser/Siliciumdioxid von 4 bis 80 und das molare Verhältnis $SiO_2/B_2O_3$ von 1,5 bis 400 ist.

4. Verfahren gemäss einem der Ansprüche 1 bis 3, in welchem die Quelle strukturierenden Mittels eine Quelle ist, die Tetraalkylammonium- oder Tetraalkylphosphonium-Kationen liefern kann.

5. Verfahren gemäss einem der Ansprüche 1 bis 3, in welchem die Quelle strukturierenden Mittels eine Quelle ist, die Kationen liefern kann, gewählt unter den Tetrapropylammonium ($TPA^+$)- und Tetrapropylphosphonium ($TPP^+$)-Kationen.

6. Verfahren gemäss einem der Ansprüche 1 bis 5, in welchem die Quelle von Fluorionen Ammoniumfluorid oder das Säurefluorid von Ammonium ist.

7. Verfahren gemäss einem der Ansprüche 1 bis 6, dadurch gekennzeichnet, dass die genannte Reaktionsmischung ausserdem umfasst ein ergänzendes Salz, gewählt aus der Gruppe gebildet durch NaCl, KCl, $NH_4Cl$ und $Na_2SO_4$, wobei das molare Verhältnis komplementäres Salz/Siliciumdioxid zwischen 0 und 4 liegt.

8. Verfahren gemäss einem der Ansprüche 1 bis 7, dadurch gekennzeichnet, dass das genannte Reaktionsgemisch Kristalle von der Art des zu erhaltenden Zeolithen enthält, wobei das Gewichtsverhältnis Zeolith-Kristalle/Siliciumdioxid zwischen 0,01 und 0,10 liegt.

9. Verfahren gemäss einem der Ansprüche 1 bis 8, dadurch gekennzeichnet, dass das Reaktionsgemisch auf eine Temperatur zwischen 60 und 230°C erhitzt wird, während einer Dauer, die im allgemeinen zwischen 8 und 400 Stunden liegt, bis zum Erhalten eines kristallinen Festkörpers, der abgetrennt wird, dann kalziniert wird bei einer Temperatur grösser 450°C, dergestalt dass die organischen Spezies, verknüpft mit dem Strukturmittel, anwesend in den Poren des Zeolithen, zerstört und ausgetrieben werden.

10. Kristallisierte Zeolithen vom Borsilikattyp, verwendet mit den Pentasilen, entsprechend der Formel:

$$(0\text{-}1)\ M_{2/n}O;\ B_2O_3;\ x\ SiO_2,$$

in welcher x variieren kann von 20 bis 1000, M Kationen der Kompensierung der Valenzen n darstellt, wobei genannte Zeolithe 0,04 bis 1,6 Gew.-% Fluor enthalten und darüber hinaus charakterisiert sind durch das Röntgenstrahlendiffraktionsdiagramm der folgenden Tabelle:

| $d_{hkl}$ (Å) | $I/I_o$ | $d_{hkl}$ (Å) | $I/I_o$ |
|---|---|---|---|
| 11,25-10,90 | FF | 3,31 -3,28 | ff |
| 10,00- 9,80 | m | 3,26 -3,22 | ff |
| 9,85- 9,61 | f | 3,16 -3,13 | ff |
| 9,10- 8,92 | ff | 3,12 -3,09 | ff |
| 7,50- 7,35 | ff | 3,05 -3,01 | ff |
| 7,13- 6,98 | ff | 2,984-2,953 | ff |
| 6,73- 6,59 | ff | 2,952-2,916 | ff |
| 6,34- 6,24 | f | 2,859-2,841 | ff |
| 6,00- 5,89 | f | 2,780-2,765 | ff |
| 5,90- 5,81 | ff | 2,728-2,708 | ff |
| 5,54- 5,44 | ff | 2,681-2,662 | ff |
| 5,37- 5,24 | ff | 2,654-2,632 | ff |
| 5,17- 5,05 | ff | 2,596-2,578 | ff |
| 5,04- 4,98 | ff | 2,562-2,541 | ff |
| 4,97- 4,91 | ff | 2,513-2,491 | ff |
| 4,89- 4,80 | ff | 2,483-2,464 | ff |
| 4,62- 4,54 | ff | 2,404-2,388 | ff |
| 4,47- 4,41 | ff | 2,392-2,372 | ff |
| 4,36- 4,30 | ff | 2,321-2,300 | ff |
| 4,27- 4,20 | f | 2,290-2,271 | ff |
| 4,09- 4,02 | ff | 2,192-2,174 | ff |
| 4,01- 3,95 | ff | 2,166-2,149 | ff |
| 3,87- 3,82 | m | 2,104-2,088 | ff |
| 3,81- 3,77 | m | 2,074-2,058 | ff |
| 3,75- 3,72 | mf | 2,061-2,048 | ff |
| 3,72- 3,67 | mf | 2,046-2,031 | ff |
| 3,65- 3,60 | f bis mf | 2,011-1,990 | f |
| 3,60- 3,56 | f bis ff | 1,985-1,966 | f |
| 3,48- 3,44 | ff | 1,946-1,928 | ff |
| 3,44- 3,40 | ff | 1,908-1,891 | ff |
| 3,39- 3,36 | ff | 1,869-1,848 | ff |
| 3,35- 3,31 | ff | | |

in welchen $d_{hkl}$ die retikuläre Äquidistanz darstellt, ausgedrückt in Angström (1 Angström (Å) = $10^{-10}$ Meter), I/Io die relative Intensität darstellt, ausgedrückt als FF = sehr stark; F = stark; mF = mittel bis stark; m = mittel; mf = mittel bis schwach; f = schwach; ff = sehr schwach.

11. Verwendung von Zeolithen gemäss Anspruch 10, als Katalysatoren oder Träger von Katalysatoren, insbesondere für die Umwandlung von Methanol in leichte Olefine, die Dehydroisomerisation von n-Butan zu Isobutylen, die Oligomerisierung von Olefinen und die katalytische Crackung.

## Claims

1. A process for synthesizing zeolites of the borosilicate type, characterized by the steps of:

a) forming a reaction medium of pH lower than 10.5, comprising mainly water, a silica source, a boron oxide source, a source of fluoride ions and a source of structurizing agent supplying organic cations,
b) heating the reaction medium formed in step a) to a temperature lower than 230°C until a crystalline compound is obtained,

c) roasting the obtained compound to a temperature higher than 450°C.

2. A process according to claim 1, characterized in that the pH of the reaction medium ranges from 1.5 to 10.5, the fluoride/silica molar ratio in said reaction medium ranges from 0.04 to 4, the organic cation/silica molar ratio ranges from 0.02 to 1, the water/silica molar ratio ranges from 3 to 200 and the $SiO_2/B_2O_3$ molar ratio ranges from 0.5 to 500.

3. A process according to claim 2, wherein the pH is from 3 to 9.5, the fluoride/silica molar ratio from 0.06 to 1.5, the organic cation/silica molar ratio from 0.06 to 0.75, the water/silica molar ratio from 4 to 80 and the $SiO_2/B_2O_3$ molar ratio from 1.5 to 400.

4. A process according to one of claims 1 to 3, wherein the source of structurizing agent is a source supplying tetraalkylammonium or tetraalkylphosphonium cations.

5. A process according to one of claims 1 to 3, wherein the source of structurizing agent is a source supplying cations selected from tetrapropylammonium ($TPA^+$) cations and tetrapropylphosponium ($TPP^+$) cations.

6. A process according to one of claims 1 to 5, wherein the source of fluoride ions is ammonium fluoride or acid ammonium fluoride.

7. A process according to one of claims 1 to 6, characterized in that the reaction mixture further comprises an additional salt selected from the group consisting of NaCl, KCl, $NH_4Cl$ and $Na_2SO_4$, the molar ration of the additional salt to silica ranging from 0 to 4.

8. A process according to one of claims 1 to 7, characterized in that said reaction mixture comprises zeolite crystals of the type to be obtained, the ratio by weight of the zeolite crystals to silica being from 0.01 to 0.10.

9. A process according to one of claims 1 to 8, characterized in that the reaction mixture is heated to a temperature from 60 to 230°C for a period generally ranging from 8 to 400 hours until a crystalline solid is obtained which is separated and then roasted at a temperature higher than 450°C, so as to destroy and remove the organic specie associated to the structurizing agent, present in the zeolithe pores.

10. Crystallized zeolites of borosilicate type, analogous to pentasils, complying with the formula:

$$(0\text{-}1)\ M_{2/n}O;\ B_2O_3;\ x\ SiO_2,$$

wherein x may range from 20 to 1000, M represents compensation cations of valence n, said zeolites containing 0.04 to 1.6% by weight of fluorine and being further characterized by the X-ray diffraction diagram of the following Table:

| $d_{hkl}$ (Å) | $I/I_o$ | $d_{hkl}$ (Å) | $I/I_o$ |
|---|---|---|---|
| 11.25-10.90 | ss | 3.31 -3.28 | ll |
| 10.00- 9.80 | m | 3.26 -3.22 | ll |
| 9.85- 9.61 | l | 3.16 -3.13 | ll |
| 9.10- 8.92 | ll | 3.12 -3.09 | ll |
| 7.50- 7.35 | ll | 3.05 -3.01 | ll |
| 7.13- 6.98 | ll | 2.984-2.953 | ll |
| 6.73- 6.59 | ll | 2.952-2.916 | ll |
| 6.34- 6.24 | l | 2.859-2.841 | ll |
| 6.00- 5.89 | l | 2.780-2.765 | ll |
| 5.90- 5.81 | ll | 2.728-2.708 | ll |
| 5.54- 5.44 | ll | 2.681-2.662 | ll |
| 5.37- 5.24 | ll | 2.654-2.632 | ll |
| 5.17- 5.05 | ll | 2.596-2.578 | ll |
| 5.04- 4.98 | ll | 2.562-2.541 | ll |
| 4.97- 4.91 | ll | 2.513-2.491 | ll |
| 4.89- 4.80 | ll | 2.483-2.464 | ll |
| 4.62- 4.54 | ll | 2.404-2.388 | ll |
| 4.47- 4.41 | ll | 2.392-2.372 | ll |
| 4.36- 4.30 | ll | 2.321-2.300 | ll |
| 4.27- 4.20 | l | 2.290-2.271 | ll |
| 4.09- 4.02 | ll | 2.192-2.174 | ll |
| 4.01- 3.95 | ll | 2.166-2.149 | ll |
| 3.87- 3.82 | m | 2.104-2.088 | ll |
| 3.81- 3.77 | m | 2.074-2.058 | ll |
| 3.75- 3.72 | ml | 2.061-2.048 | ll |
| 3.72- 3.67 | ml | 2.046-2.031 | ll |
| 3.65- 3.60 | l to ml | 2.011-1.990 | l |
| 3.60- 3.56 | l to ll | 1.985-1.966 | l |
| 3.48- 3.44 | ll | 1.946-1.928 | ll |
| 3.44- 3.40 | ll | 1.908-1.891 | ll |
| 3.39- 3.36 | ll | 1.869-1.848 | ll |
| 3.35- 3.31 | ll | | |

wherein $d_{hkl}$ is the reticular equidistance expressed as angströms (1 angström = $10^{-10}$m), I/Io is the relative intensity expressed as ss = very strong, s = strong, ml = medium to strong, m = medium, ml = medium to low, l = low, ll = very low.

11. The use of zeolites according to claim 10 as catalysts or catalyst carriers, particularly in the conversion of methanol to light olefins, the dehydroisomerisation of n-butane to isobutylene, the oligomerisation of olefins and the catalytic cracking.